(19) Europäisches Patentamt
European Patent Office
Office européen des brevets

(11) **EP 1 461 011 B1**

(12) **FASCICULE DE BREVET EUROPEEN**

(45) Date de publication et mention de la délivrance du brevet:
**11.03.2009  Bulletin 2009/11**

(21) Numéro de dépôt: **03709852.2**

(22) Date de dépôt: **03.01.2003**

(51) Int Cl.:
*A61K 8/97* *(2006.01)*      *A61Q 19/08* *(2006.01)*

(86) Numéro de dépôt international:
**PCT/FR2003/000005**

(87) Numéro de publication internationale:
**WO 2003/059243 (24.07.2003 Gazette 2003/30)**

(54) **COMPOSITION COSMETIQUE POUR LUTTER CONTRE LE VIEILLISSEMENT CUTANE**

KOSMETISCHE ZUSAMMENSETZUNG GEGEN DAS ALTERN DER HAUT

COSMETIC COMPOSITION FOR TREATMENT AGAINST SKIN AGEING

(84) Etats contractants désignés:
**AT BE BG CH CY CZ DE DK EE ES FI FR GB GR HU IE IT LI LU MC NL PT SE SI SK TR**

(30) Priorité: **03.01.2002  FR 0200051**
**08.07.2002  FR 0208566**

(43) Date de publication de la demande:
**29.09.2004  Bulletin 2004/40**

(73) Titulaire: **Laboratoires Clarins**
**92200 Neuilly sur Seine (FR)**

(72) Inventeur: **COURTIN, Olivier**
**F-92100 Boulogne-sur-Seine (FR)**

(74) Mandataire: **Breese, Pierre**
**BREDEMA**
**38, avenue de l'Opéra**
**75002 Paris (FR)**

(56) Documents cités:
**FR-A- 2 805 464**

- **DATABASE BIOSIS [en ligne] BIOSCIENCES INFORMATION SERVICE, PHILADELPHIA, PA, US; août 1998 (1998-08) SEVERIEN BARIGAH TETE ET AL: "Growth and net assimilation rate of seedlings of ten Guianan tree species grown under five light regimes." Database accession no. PREV199900033797 XP002214960 & ANNALES DES SCIENCES FORESTIERES (PARIS), vol. 55, no. 6, août 1998 (1998-08), pages 681-706, ISSN: 0003-4312**

**EP 1 461 011 B1**

**Description**

L'invention concerne le domaine des produits cosmétiques et vise à offrir une nouvelle composition cosmétique qui permet de lutter contre le vieillissement cutané contenant un extrait, notamment hydrosoluble, de *Bocoa prouacensis.*

Par « composition cosmétique capable de lutter contre le vieillissement cutané », on entend toute composition cosmétique capable d'empêcher et/ou de limiter l'apparition des signes du vieillissement cutané tels que ridules, rides et peau sèche. On entend également toute composition cosmétique capable, lorsque la peau présente déjà des signes d'un vieillissement cutané, de corriger et/ou d'amoindrir ces signes de vieillissement.

Il existe deux types de vieillissement cutané, le vieillissement intrinsèque ou chronologique induit par des processus génétiquement programmés et le vieillissement actinique lié aux expositions solaires. Ce dernier type de vieillissement est également appelé héliodermie.

Le vieillissement cutané se caractérise par l'amincissement de l'épaisseur de la peau, avec une perte de 6% de l'épaisseur de la peau dès la naissance tous les dix ans (Robert et al., 2001, J. Méd. Esth. et Chir. Derm., vol. 28, pages 27-35). En effet, la prolifération des kératinocytes de l'épiderme ralentit au cours du temps ce qui provoque un amincissement tissulaire et une altération de la fonction de barrière.

Au niveau du derme, le métabolisme des fibroblastes ralentit également avec l'âge et leur capacité à synthétiser la matrice extracellulaire diminue ce qui provoque une atrophie du derme (Benoît et al., 2000, IFSCC Magazine, vol. 3, pages 11-17).

Cette modification quantitative s'accompagne d'une modification qualitative de la composition de cette matrice extracellulaire. La quantité de collagène décline avec l'âge (Shuster et al., 1975, Brit. J. Dermatol., vol. 93, pages 639-43). En effet, la synthèse des collagènes fibrillaires (de type I et III en particulier) diminue légèrement avec l'âge mais de façon moindre pour le collagène de type III. La synthèse d'élastine et de fibronectine augmente avec l'âge (Robert et al., 2001, J. Méd. Esth. et Chir. Derm., vol. 28, pages 27-35). Parallèlement, la concentration effective de protéoglycanes et de glycosaminoglycanes dans le derme diminue, ce fait est particulièrement net pour l'acide hyaluronique qui devient indétectable à partir de 60 ans (Alirezai et Meynadier, 1997, Nouv. Dermatol., vol. 16, pages 41-47). Cette modification qualitative de la composition de la matrice extracellulaire cutanée modifie les propriétés mécaniques de la peau dont l'élasticité diminue et qui devient moins résistante à la pression. Cette modification se manifeste également par une perte d'hydratation des tissus.

La modification qualitative de la composition de la matrice extracellulaire cutanée est aggravée et accélérée par l'activité catabolique liée à l'âge. En effet, toutes les cellules de l'organisme sont capables de produire des enzymes protéolytiques pouvant s'attaquer à certains des constituants de la matrice extracellulaire comme les fibres de collagène et d'élastine. Ces enzymes sont respectivement appelées collagénases et élastases. Lors du vieillissement chronologique, une augmentation de l'activité élastasique des fibroblastes a pu être mis en évidence *in vitro* (Pelletier-Lebon et al., 1989, Cutaneous development, aging and repair, vol. 18, pages 341-345, Fidia Research Series, Liviana Press). Cette augmentation semble être un processus intrinsèque au niveau de la régulation des gènes et de leurs transcrits.

L'augmentation de l'activité de ces protéases entraîne une augmentation de la libération de peptides pouvant agir sur les cellules et modifier leur phénotype. Par exemple, le récepteur de l'élastine activé par des peptides de l'élastine augmente la production d'élastases par les fibroblastes (Archilla-Marcos et Robert, 1993, Clin. Physiol. Biochem., vol. 10, pages 86-91).

Le vieillissement actinique est induit par l'accumulation de radicaux libres dérivés de l'oxygène au niveau de la peau. Cet excès de radicaux libres ne peut plus être neutralisé par les défenses anti-radicalaires endogènes et entraîne des modifications chimiques des constituants cellulaires et extracellulaires (Benoît et al., 2000, IFSCC Magazine, vol. 3, pages 11-17). Lors du vieillissement actinique, d'importantes modifications enzymatiques conditionnent la dégradation de la matrice extracellulaire du derme. En effet, les réactions induites par les radicaux libres dérivés de l'oxygène dégradent notamment le collagène dont les molécules sont coupées et libèrent des peptides activateurs des polynucléaires, ces derniers accentuant la dégradation du tissu conjonctif (Alirezai et Meynadier, 1997, Nouv. Dermatol., vol. 16, pages 41-47). De plus, l'activité élastasique de la peau augmente à la suite d'irradiations par les UV (Labat-Robert et al., 2000, J. Photochem. Photobiol., vol. 57, pages 113-118).

Ainsi, une composition cosmétique utile pour lutter contre le vieillissement cutané doit contenir des inhibiteurs de protéases et plus particulièrement des inhibiteurs de collagénases et d'élastases, et des actifs anti-radicalaires.

Les travaux de la Demanderesse ont permis de mettre en évidence qu'un extrait hydrosoluble de *Bocoa prouacensis* présentait à la fois une activité anti-collagénase et une activité anti-radicalaire qui font de cet extrait un composant utile pour lutter contre le vieillissement cutané.

Le *Bocoa prouacensis* est également appelé « boco » ou « arbre de fer ». Cet arbre de la famille des Caesalpiniaceae est un arbre de forêt primaire présentant une croissance exponentielle lente, un bois d'une très grande densité (d'où son nom de bois de fer) et une longévité remarquable. La répartition géographique des populations de *Bocoa prouacensis* couvre le tiers nord du département de la Guyane ainsi que la région nord-est du Surinam voisin. Il s'agit d'une donc d'une espèce remarquable endémique du bouclier des Guyanes.

La densité, les propriétés mécaniques et la faible minéralisation du duramen de cet arbre en font un matériau de grande valeur en ébénisterie, marqueterie, lutherie et bijouterie. De plus, les feuilles de *Bocoa prouacensis* sont utilisées traditionnellement par les Samaracas pour leurs propriétés fortifiantes, stimulantes, défatigantes voire aphrodisiaques. Les feuilles rentrent notamment dans la préparation d'une décoction utilisée en bain ou en douche par les Samaracas.

La composition cosmétique objet de la présente invention contient un extrait de *Bocoa prouacensis.*

Dans un premier mode de réalisation de la présente invention, l'extrait de *Bocoa prouacensis* est un extrait issu de feuilles de *Bocoa prouacensis.* De préférence, il s'agit d'un extrait hydrosoluble.

L'extrait hydrosoluble et plus particulièrement hydroglycolique de feuilles de *Bocoa prouacensis* utilisé dans la composition selon l'invention a un aspect liquide limpide, une couleur ambrée foncée et une odeur forte. Il présente les caractéristiques analytiques suivantes :

- matières sèches = 22 g/l,
- polyphénols = 1250 mg/ml
- pH = 5,9.

Dans un second mode de réalisation de la présente invention, l'extrait de *Bocoa prouacensis* est un extrait issu de bois et/ou d'écorce de *Bocoa prouacensis.* De préférence, il s'agit d'un extrait hydrosoluble. Par « bois », on entend aussi bien le bois d'aubier, le bois de coeur qu'un mélange de ceux-ci.

L'extrait hydrosoluble et plus particulièrement hydroglycolique issu de bois et/ou d'écorce de *Bocoa prouacensis* utilisé dans la composition selon l'invention présente les caractéristiques analytiques suivantes:

- matières sèches = 19,3 g/l,
- polyphénols = 2600 mg/ml
- pH = 4,8.

Dans un troisième mode de réalisation de la présente invention, l'extrait de *Bocoa prouacensis* est un mélange de l'extrait issu de feuilles de *Bocoa prouacensis* tel que défini ci-dessus et de l'extrait issu de bois et/ou d'écorce de *Bocoa prouacensis* tel que défini ci-dessus. Dans un mélange selon l'invention, le rapport entre l'extrait issu de feuilles et de l'extrait issu de bois et/ou d'écorce se situe dans 99,9 : 0,1 et 0,1 : 99,9 (vol : vol).

Dans la présente invention, on entend par « extrait hydrosoluble de *Bocoa prouacensis* », aussi bien l'extrait issu de feuilles de *Bocoa prouacensis,* l'extrait issu de bois et/ou d'écorce de *Bocoa prouacensis* que le mélange des deux extraits tels que définis ci-dessus.

Ainsi, la composition cosmétique objet de la présente invention contient de l'ordre de 0,1 à 10 % en poids et de préférence de 0,5 à 5 % en poids d'extrait hydrosoluble de *Bocoa prouacensis.*

La composition cosmétique objet de la présente invention peut avantageusement contenir en outre au moins un composé ou extrait végétal capable d'augmenter l'hydratation et/ou l'élasticité de la peau. Ces composés ou extraits végétaux sont avantageusement choisis dans le groupe constitué par de l'huile de Péqui (*Caryoca brasiliens* ou *Caryoca corieaceum*), un extrait hydrosoluble de *Mourera fluviatilis,* un extrait hydrosoluble d'anthyllis (*Anthyllis vulneraria),* un extrait hydrosoluble de *Pinus Lambertiana*, un extrait de houblon, du beurre de karité.

L'arbre fruitier du nom commun de Péqui (*Caryoca brasiliens* ou *Caryoca corieaceum*) appartient à la famille des Caryocaceae qui comporte 20 espèces disséminées dans l'Amazonie du Nord et du Nord-Ouest du Brésil. L'huile de Péqui est obtenue par pression à froid de la pulpe du fruit de Péqui. Les acides gras présents en majorité dans cette huile sont des acides gras mono-insaturés du type oléique. Cette huile, comme toutes les huiles végétales, apporte douceur, corps et texture aux crèmes et lotions cosmétiques. De plus, elle protège la peau contre la déshydratation. C'est pour cette activité qu'elle est préférentiellement utilisée dans la composition objet de la présente invention.

Un extrait hydrosoluble de *Mourera fluviatilis* (plante aquatique poussant dans les eaux tumultueuses des rivières guyanaises) utilisable dans la composition cosmétique selon l'invention est obtenu en broyant la plante entière sans les racines. Une extraction aqueuse est ensuite réalisée. L'extrait de *Mourera fluviatilis* est obtenu après une étape de purification/ filtration. Cet extrait agit spécifiquement sur les couches cornées épidermiques et exerce à la fois un effet régulateur d'hygroscopicité, un effet hydrorégulateur substantif, un effet ralentisseur de déshydratation et un effet prolongateur d'hydratation. Dans le cadre de la présente invention, il permet de freiner la déshydratation cutanée induite par le vieillissement.

L'extrait hydrosoluble d'anthyllis (*Anthyllis vulneraria*) utilisé dans les compositions de l'invention est obtenu à partir de fleurs et, plus particulièrement, de fleurs séchées. Les fleurs séchées sont broyées, la poudre de fleurs ainsi obtenue est solubilisée dans de l'eau. La solution subit une hydrolyse enzymatique à l'aide d'une protéase avant d'être mise à décanter. La phase soluble contenant les molécules actives est concentrée avant de subir une filtration stérilisante.

L'extrait hydrosoluble d'anthyllis (*Anthyllis vulneraria*) ainsi obtenu est utilisable dans la composition cosmétique selon l'invention. En effet, cet extrait permet d'augmenter la synthèse des glycosaminoglycanes et protéoglycanes et stimule donc les molécules responsables de l'hydratation de la peau.

Le *Pinus Lambertiana* est un pin de Nouvelle-Zélande dont un extrait du tronc et des feuilles est très riche en cyclidol (D-pintol). Il s'agit d'un sucre constituant un oligosaccharide majeur qui contribue à la survie de certaines espèces soumises à un stress salin. Ce sucre présente aussi des propriétés stabilisatrices des membranes qui confèrent à l'extrait hydrosoluble de *Pinus Lambertiana* des propriétés hydratantes au niveau cutané utilisées dans la composition selon l'invention.

L'extrait de houblon utilisable dans la composition cosmétique selon l'invention permet l'apport de fraction insaponifiable, constituée par l'ensemble des éléments naturels de la plante extraits après saponification. Cet extrait est donc riche en tocophérol, en phytostérols, en alcools terpéniques et en caroténoïdes. Les phytostérols sont des précurseurs de la vitamine D qui contribue à l'amélioration de la croissance et de la différenciation cellulaire. La vitamine D étant instable et dégradée par l'oxygène et surtout par la lumière, il est préférable d'utiliser ses précurseurs que sont les phytostérols. Par conséquent, l'extrait de houblon présente les propriétés suivantes : stimulation du métabolisme cutané, accélération des processus de régénération et lutte contre la sénescence et le dessèchement cutané. De la même façon, le beurre de karité qui est utilisable dans la composition cosmétique selon l'invention est riche en insaponifiables et en phytostérols. Par conséquent, il protège la peau contre le vieillissement cutané et possède une action trophique en améliorant l'aspect des peaux sèches et vieillies.

La composition objet de la présente invention peut également contenir tout extrait végétal riche en flavonoïdes et/ou en tanins. En effet, les flavonoïdes interagissent avec les fibres de collagène et d'élastine et les protègent très efficacement de la dégradation protéolytique présente lors du vieillissement cutané. De plus, la richesse en tanins des extraits végétaux leur confèrent des activités stimulante, anti-oxydante et protectrice. Une activité anti-oxydante est particulièrement utile pour lutter contre le vieillissement cutané. A titre d'exemple et de façon non exhaustive, on peut citer comme extrait végétal riche en tanins utilisable dans le cadre de la présente invention l'extrait hydroglycolique de cardère sauvage (*Dipsacus sylvestris* Mill.). Le cardère sauvage également appelé « Peigne-de-loup », « Baignoire de Vénus » ou « Cabaret-des-oiseaux » est une plante herbacée bisannuelle de 60 à 150 cm que l'on trouve partout en Europe au bord des chemins et des fossés. L'extrait hydroglycolique de cardère sauvage est obtenu par digestion dans le mélange hydroglycolique à partir de la partie aérienne de la plante finement broyée, puis il est décoloré, clarifié, standardisé et conservé.

Les extraits végétaux riches en flavonoïdes utilisables sont avantageusement choisis dans le groupe constitué par un extrait hydrosoluble de romarin (*Rosmarinus officinalis*) et un extrait hydrosoluble de galanga (*Alpinia officinarum*).

Un extrait hydrosoluble de romarin (*Rosmarinus officinalis*) est obtenu à partir des feuilles de cette plante commune de la région méditerranéenne. Cet extrait contient des flavonoïdes, de l'acide ursolique et de l'acide rosmarinique.

Le galanga (*Alpinia officinarum*) de la famille des Zingibéracées est un arbrisseau originaire de l'Est de l'Asie dont le rhizome noir et noueux ressemble à celui du gingembre. L'extrait hydrosoluble de galanga utilisé dans les compositions de l'invention est un extrait contenant des flavonoïdes. Cet extrait est avantageusement préparé à partir des rhizomes de galanga. L'extrait de galanga est obtenu par extraction contrôlée par le mélange eau/ propylène glycol suivie d'une étape de centrifugation/ filtration. Cet extrait de galanga est riche en phytostérols et flavonoïdes (1,5 g/l équivalent rutine) tels que la galangine et le kaempférol et en huiles essentielles comme le bornéol, le cinéole, le caryophyllène et le géraniol.

Les compositions selon l'invention peuvent encore comprendre un ou plusieurs agents de formulation ou additifs d'usage connu et classique dans les compositions cosmétiques et dermatologiques telles que, à titre d'exemple et de façon non limitative, des adoucissants, des colorants, des actifs filmogènes, des tensioactifs, des parfums, des conservateurs, des émulsionnants, des huiles, des glycols, des agents absorbeurs de sébum (talc), des vitamines comme la vitamine E, des filtres UV, etc.... Grâce à ces connaissances en matière de cosmétiques, l'homme du métier saura quels agents de formulation ajouter aux compositions de l'invention et en quelles quantités en fonction des propriétés recherchées.

Il faut remarquer que l'ajout de filtres UV dans la composition cosmétique selon l'invention permet de lutter contre le vieillissement actinique induit par les UV. Les filtres UV utilisables dans la composition cosmétique selon l'invention peuvent être des filtres chimiques ou minéraux. De façon avantageuse, les filtres UV utilisables dans la composition cosmétique selon l'invention sont les filtres choisis dans le groupe constitué par tous les pigments inorganiques, tels que les oxydes de titane et les oxydes de zinc, le butyl méthoxy dibenzoyl méthane, l'octyl methoxycinnamate, l'octyl salicylate, l'octocrylène, la benzophénone-3, l'acide 2-phényl benzimidazole-O-sulphonique, le salicylate d'homomenthyle, le 3-(4-méthyl benzylidène) camphre et l'octyl triazone.

De plus, la vitamine E, également connue sous le nom d'acétate de tocophérol, utilisable dans la composition selon l'invention prévient du vieillissement cutané. En effet, cette vitamine est un anti-oxydant naturel qui protège la peau des effets nocifs des radicaux libres produits notamment par les rayons UVB en bloquant la péroxydation lipidique.

Elle améliore également l'élasticité cutanée et la capacité de la peau à retenir l'eau ce qui entraîne une diminution des rides et de l'aspect rêche.

Les compositions selon l'invention peuvent se présenter sous toute forme connue de l'homme du métier dans le domaine de la cosmétologie et de la dermatologie sans autre restriction galénique que l'application sur le visage et sur le corps. De façon avantageuse, les compositions selon l'invention se présentent sous la forme d'un gel, d'une lotion, d'une crème, d'une émulsion, d'un lait, d'un spray, etc....

La présente invention concerne également l'utilisation cosmétique d'un extrait de *Bocoa prouacensis* pour lutter contre le vieillissement cutané et l'utilisation d'un extrait de *Bocoa prouacensis* pour la préparation d'une composition cosmétique ou dermatologique pour le soin de la peau. L'activité anti-radicalaire de l'extrait de *Bocoa prouacensis* permet son utilisation ou l'utilisation d'une composition le contenant pour lutter contre le vieillissement actinique. L'activité anti-collagénase de l'extrait de *Bocoa prouacensis* permet son utilisation ou l'utilisation d'une composition le contenant pour lutter contre le vieillissement intrinsèque.

D'autres avantages et caractéristiques de l'invention apparaîtront à la lecture des exemples qui suivent concernant l'activité anti-radicalaire et anti-collagénase de l'extrait hydroglycolique de *Bocoa prouacensis* et quelques exemples de formulation qui sont donnés à titre illustratif et ne sauraient être interprétés comme limitant la portée de l'invention.

1. Préparation et caractéristiques de l'extrait hydrosoluble de *Bocoa prouacensis.*

1. Préparation et caractéristiques de l'extrait de feuilles de *Bocoa prouacensis.*

L'extrait hydrosoluble de feuilles de *Bocoa prouacensis* mis en oeuvre dans les compositions de l'invention peut être obtenu de la façon suivante :

i) Solubilisation dans un milieu eau / alcool. De préférence eau (75%) / alcool (25%).
ii) Traitement thermique durant 1 à 4 heures entre 40 et 80°C.
iii) Séparation des phases solubles et insolubles par filtration, décantation ou centrifugation.
iv) Filtration stérilisante sur membrane 0,45 $\mu$m.

Le ratio plante / extrait est de 0,6 à 0,8 1 d'actif.

La caractérisation analytiques l'extrait comprend de 800 à 2000 $\mu$m /ml polyphénols totaux dont :

- flavone (apigénine) 15 à 25%, et
- flavonol (kaempférol) 20 à 25%.

Les caractéristiques bactériologiques de l'extrait sont :

- Flore mésophile total < 100 germes/g
- Aucun germe pathologique présent.

L'extrait est stocké de préférence à 4°C. Il est soluble dans un milieu aqueux et dans l'éthanol (60/40 éthanol/en (v/v)). Il est stable à des températures de plus de 60°C pendant au moins deux heures et est compatible avec un pH inférieur ou égal à 6.

L'extrait n'est pas irritant selon les tests d'irritation de la peau et des yeux. Il n'entraîne pas de réaction de sensibilité ou d'allergie selon le test de Magnusson et Kligman. Il n'est pas phototoxique et non mutagène selon le test d'Ames.

2. Préparation et caractéristiques de l'extrait de bois et/ou d'écorce de *Bocoa prouacensis.*

L'extrait hydrosoluble de bois et/ou d'écorce de *Bocoa prouacensis* mis en oeuvre dans les compositions de l'invention peut être obtenu de la façon suivante :

i) Solubilisation dans un milieu eau / butylène glycol (50/50 - v/v),
ii) Séparation des phases solubles et insolubles par filtration,
iii) Filtration stérilisante.

Le ratio plante / extrait est de 1/2.

II. Activité anti-radicalaire de l'extrait hydrosoluble de *Bocoa prouacensis.*

1. Principe du dosage.

Le DiPhényl picrylhydrazyl hydrate (DPPH; Sigma D9132) est un radical libre, absorbant dans le violet à 517 nm.
Un produit anti-radicalaire entraîne une disparition de la coloration violette.

2. Protocole expérimental.

a. Préparation des solutions.

La solution DPPH est préparée en dissolvant 4,8 mg de DPPH dans 200 ml de méthanol (Prolabo Rectapur 20846.292).
Les échantillons à tester sont dilués de manière à obtenir une concentration totale d'environ $50\mu$ g/ml. Les échantillons à tester sont un extrait de *Bocoa prouacensis* à 1%, un extrait de *Bocoa prouacensis* à 2%, un extrait de *Bocoa prouacensis* à 5% et un extrait de *Bocoa prouacensis* à 10%, l'extrait de *Bocoa prouacensis* étant soit un extrait de feuilles, soit un extrait de bois et/ou d'écorce.
Le témoin contrôle est un échantillon dont l'activité anti-radicalaire est connue. Il s'agit de l'hélioxine diluée au 1/20 dans de l'eau distillée.

b. Dosage.

Dans des tubes à hémolyse, on met 2,5 ml de solution DPPH puis on ajoute :

- soit 200 $\mu$l de la solution diluée du produit à tester,
- soit 200 $\mu$l d'eau distillée (pour le blanc),
- soit 200 $\mu$l du témoin contrôle.

On agite les mélanges puis on attend 20 min avant de lire les densités optiques à 517 nm contre l'air dans un spectrophotomètre.

c. Résultats.

Le résultat est fourni en pourcentage d'activité anti-radicalaire selon la formule suivante: % activité = [(DO blanc - DO produit)/ DO blanc] x 100
Le tableau 1 ci-après présente les résultats obtenus.

Tableau 1: Effet de différentes concentrations d'extrait de *Bocoa prouacensis* sur l'activité anti-radicalaire.

|  | Activité anti-radicalaire | |
|---|---|---|
|  | Extrait de feuilles | Extrait de bois et/ou d'écorce |
| Extrait de *Bocoa prouacensis* à 1% | 19% | 50% |
| Extrait de *Bocoa prouacensis* à 2% | 36% | 78% |
| Extrait de *Bocoa prouacensis* à 5% | 73% | 81% |
| Extrait de *Bocoa prouacensis* à 10% | 82% | 83% |

L'extrait hydrosoluble de feuilles ou de bois et/ou d'écorce de *Bocoa prouacensis* possède une activité anti-radicalaire et cet effet est dose-dépendant.
De plus, l'extrait de bois et/ou d'écorce possède une activité anti-radicalaire supérieure à l'extrait de feuilles de *Bocoa prouacensis.* L'échantillon à 1% d'extrait de bois et/ou d'écorce présente une activité anti-radicalaire de 50%.

III. Activité anti-collagénase de l'extrait hydrosoluble de *Bocoa prouacensis.*

1. Principe du dosage.

Une unité de collagénase hydrolyse 1 μmole de FALGPA (Furylacryloyl-Leu-Gly-Pro-Ala; Sigma F 5135) par minute à 25°C à pH 7,5 en présence d'ions calcium. Cette réaction d'hydrolyse se traduit par une diminution de l'absorbance à 324 nm.

Si un produit possède une activité anti-collagénase, la réaction sur le substrat FALGPA est inhibée et l'absorbance ne diminue pas.

2. Protocole expérimental.

a. Préparation des solutions.

La solution tampon est obtenue en dissolvant, dans 1 l d'eau distillée, 8,96 g de tricine (Sigma T 7911), 23,4 g de chlorure de sodium (Merck 1 06404.1000) et 1,47 g de chlorure de calcium (Cooper 93 042).

La solution substrat FALGPA à 0,105 mM est obtenue en mélangeant 5 mg de FALGPA dans 100 ml de solution tampon. La solution obtenue est agitée pendant au moins 30 min, puis aliquotée en fractions d'environ 15 ml qui sont congelées.

La solution collagénase (Sigma C 7657) est une solution à 5 U/ml congelée en aliquotes de 200 μl.

Les échantillons à tester sont un extrait de *Bocoa prouacensis* à 1%, un extrait de *Bocoa prouacensis* à 2%, un extrait de *Bocoa prouacensis* à 5% et un extrait de *Bocoa prouacensis* à 10%, l'extrait de *Bocoa prouacensis* étant soit un extrait de feuilles, soit un extrait de bois et/ou d'écorce.

b. Dosage.

La solution substrat FALGPA et la solution collagénase sont décongelées.

Dans une cuve en quartz, sont mis 2 ml de solution substrat FALGPA avec

- soit 400 μl d'échantillon à tester,
- soit 400 μl d'eau distillée (pour le blanc).

On agite les mélanges puis les densités optiques à 324 nm sont lues dans un spectrophotomètre.

L'ajout de 20 μl de solution collagénase déclenche la réaction. Les densités optiques doivent être lues 10 min après cet ajout.

c. Résultats.

Le résultat est fourni en pourcentage d'activité anti-collagénase des échantillons testés selon la formule suivante :

```
% activité = [(ΔDO blanc - ΔDO échantillon)/ΔDO blanc] x 100
```

avec

```
ΔDO = [DO (à t0) - DO (à t10 min)]/ 10
```

Le tableau 2 ci-dessous présente les résultats obtenus.

Tableau 2: Effet de différentes concentrations d'extrait de *Bocoa prouacensis* sur l'activité anti-collagénase.

| | Activité anti-collagénase | |
|---|---|---|
| | Extrait de feuilles | Extrait de bois et/ou d'écorce |
| Extrait de *Bocoa prouacensis* à 1 % | 16% | 22% |

(suite)

|  | Activité anti-collagénase | |
|---|---|---|
|  | Extrait de feuilles | Extrait de bois et/ou d'écorce |
| Extrait de *Bocoa prouacensis* à 2% | 26% | 36% |
| Extrait de *Bocoa prouacensis* à 5% | 58% | 49% |
| Extrait de *Bocoa prouacensis à* 10% | 76% | 74% |

L'extrait hydrosoluble de *Bocoa prouacensis* possède une activité anti-collagénase et cet effet est dose-dépendant. L'extrait de feuilles ou l'extrait de bois et/ou d'écorce de *Bocoa prouacensis* ont une activité anti-collagénase comparable.

IV. Exemples de formules quantitatives de composition de l'invention.

1) Crème visage.

|  | % |
|---|---|
| GLYCERINE | 4,0 |
| AGENT SÉQUESTRANT | 0,1 |
| CONSERVATEURS | 1,0 |
| AGENTS EPAISSISSANTS | 1,0 |
| TALC | 2,5 |
| ALCOOL GRAS | 1,0 |
| EMULSIONNANT | 6,5 |
| TRIGLYCÉRIDES | 1,0 |
| ESTER D'ACIDE GRAS | 5,0 |
| SUCRES | 6,0 |
| HUILES DE SILICONE | 5,5 |
| EXTRAIT D'ALOES | 1,0 |
| FILTRES SOLAIRES UVA/UVB | 5,0 |
| EXTRAIT DE BOCOA PROUACENSIS | 3,0 |
| EXTRAIT DE MOURERA FLUVIATILIS | 2,0 |
| HUILE DE PEQUI | 2,0 |
| BEURRE DE KARITÉ | 2,0 |
| EXTRAIT DE HOUBLON | 1,0 |
| EXTRAIT DE PINUS LAMBERTIANA | 1,0 |
| PARFUM | 0,5 |
| EAU PURIFIÉE | *q.s.p.*   *100* |

2) Gel visage.

|  | % |
|---|---|
| GLYCOL | 2,0 |
| AGENT EPAISSISSANT NEUTRALISÉ | 1,0 |
| AGENT SÉQUESTRANT | 0,1 |
| CONSERVATEURS | 0,7 |
| ALCOOL ETHYLIQUE | 5,0 |
| EXTRAIT DE BOCOA PROUACENSIS | 3,0 |

(suite)

|  |  | % |
|---|---|---|
| EXTRAIT DE MOURERA FLUVIATILIS |  | 2,0 |
| EXTRAIT DE CARDERE |  | 2,0 |
| EXTRAIT DE ROMARIN |  | 2,0 |
| EXTRAIT D'ANTHYLIS |  | 1,0 |
| SOLUBILISANT |  | 1,0 |
| PARFUM |  | 0,5 |
| EAU PURIFIÉE | q.s.p. | 100 |

3) Sérum visage.

|  |  | % |
|---|---|---|
| PROPYLÈNE GLYCOL |  | 2,0 |
| BUTYLENE GLYCOL |  | 4,0 |
| AGENT SEQUESTRANT |  | 0,1 |
| CONSERVATEUR |  | 0,6 |
| C.M.C |  | 0,4 |
| EXTRAIT DE BOCOA PROUACENSIS |  | 4,0 |
| EXTRAIT DE MOURERA FLUVIATILIS |  | 2,0 |
| EXTRAIT DE CARDERE |  | 2,0 |
| EXTRAIT DE PINUS LAMBERTIANA |  | 2,0 |
| EXTRAIT DE HOUBLON |  | 2,0 |
| EXTRAIT DE ROMARIN |  | 2,0 |
| PARFUM |  | 0,2 |
| SOLUBILISANT |  | 0,8 |
| EAU FLORALE |  | 20,0 |
| EAU PURIFIÉE | q.s.p. | 100 |

**Revendications**

1. Composition cosmétique contenant un extrait hydrosoluble de *Bocoa prouacensis.*

2. Composition cosmétique selon la revendication 1, **caractérisée en ce que** ledit extrait est choisi parmi un extrait issu de feuilles de *Bocoa prouacensis,* un extrait issu de bois et/ou d'écorce de *Bocoa prouacensis* et un mélange de ceux-ci.

3. Composition cosmétique selon l'une quelconque des revendications 1 ou 2, **caractérisée en ce qu'**elle contient de l'ordre de 0,1 à 10 % en poids et de préférence de 0,5 à 5 % en poids d'extrait hydrosoluble de *Bocoa prouacensis.*

4. Composition cosmétique selon l'une quelconque des revendications 1 à 3, **caractérisée en ce qu'**elle contient au moins un composé ou extrait végétal capable d'augmenter l'hydratation et/ou l'élasticité de la peau.

5. Composition cosmétique selon l'une quelconque des revendications 1 à 4, **caractérisée en ce qu'**elle contient au moins un extrait végétal riche en flavonoïdes et/ou en tanins.

6. Composition cosmétique selon l'une quelconque des revendications précédentes, **caractérisée en ce qu'**elle comprend un ou plusieurs agents de formulation ou additifs tels que des adoucissants, des colorants, des actifs filmogènes, des tensioactifs, des parfums, des conservateurs, des émulsionnants, des huiles, des glycols, des agents absorbeurs de sébum, des vitamines et des filtres UV.

9

7. Utilisation cosmétique d'un extrait de *Bocoa prouacensis* pour lutter contre le vieillissement cutané.

8. Utilisation d'un extrait de *Bocoa prouacensis* pour la préparation d'une composition cosmétique ou dermatologique pour le soin de la peau.

9. Utilisation selon l'une des revendications 7 ou 8, **caractérisée en ce que** ledit extrait de *Bocoa prouacensis* ou la composition le contenant sont destinés à lutter contre le vieillissement actinique.

10. Utilisation selon l'une des revendications 7 ou 8, **caractérisée en ce que** ledit extrait de *Bocoa prouacensis* ou la composition le contenant sont destinés à lutter contre le vieillissement intrinsèque.


**Claims**

1. Cosmetic composition containing a hydrosoluble extract of *Bocoa prouacensis.*

2. Cosmetic composition according to claim 1, **characterised in that** said extract is selected from an extract of *Bocoa prouacensis* leaves, an extract of wood and/or bark of *Bocoa prouacensis* and a mixture thereof.

3. Cosmetic composition according to any one of claims 1 or 2, **characterised in that** it contains around 0.1 to 10% in weight and preferably 0.5 to 5% in weight of the hydrosoluble extract of *Bocoa prouacensis.*

4. Cosmetic composition according to any one of claims 1 to 3, **characterised in that** it contains at least one compound or vegetable extract that is capable of increasing the hydration and/or elasticity of the skin.

5. Cosmetic composition according to any one of claims 1 to 4, **characterised in that** it contains at least one vegetable extract that is rich in flavonoids and/or tannins.

6. Cosmetic composition according to any one of the previous claims, **characterised in that** it contains one or several additives or formulation agents such as softeners, colorants, filmogen active agents, surface active agents, perfumes, preservatives, emulsifiers, oils, glycols, sebum-absorbing agents, vitamins and UV filters.

7. Cosmetic use of an extract of *Bocoa prouacensis* to combat ageing of the skin.

8. Use of an extract of *Bocoa prouacensis* for the preparation of a cosmetic or dermatological composition for skin care.

9. Use according to one of claims 7 or 8, **characterised in that** said extract of *Bocoa prouacensis* or the composition containing it are designed to combat actinic ageing.

10. Use according to one of claims 7 or 8, **characterised in that** said extract of *Bocoa prouacensis* or the composition containing it are designed to combat intrinsic ageing.


**Patentansprüche**

1. Kosmetische Zusammensetzung, umfassend einen wasserlöslichen Extrakt aus Bocoa prouacensis.

2. Kosmetische Zusammensetzung nach Anspruch 1, **dadurch gekennzeichnet, dass** der Extrakt aus einem Extrakt, der aus Blättern des Bocoa prouacensis stammt, einem Extrakt, der aus dem Holz und / oder der Rinde des Bocoa prouacensis stammt, und einer Mischung aus eben diesen gewählt wird.

3. Kosmetische Zusammensetzung nach einem der Ansprüche 1 oder 2, **dadurch gekennzeichnet, dass** sie einen wasserlöslichen Extrakt aus Bocoa prouacensis in der Höhe von 0,1 bis 10 Gew.-% und vorzugsweise von 0,5 bis 5 Gew.-% enthält.

4. Kosmetische Zusammensetzung nach einem der Ansprüche 1 bis 3, **dadurch gekennzeichnet, dass** sie mindestens eine pflanzliche Verbindung oder einen Pflanzenextrakt enthält, die imstande sind, die Feuchtigkeitsversorgung und / oder die Elastizität der Haut zu erhöhen.

**5.** Kosmetische Zusammensetzung nach einem der Ansprüche 1 bis 4, **dadurch gekennzeichnet, dass** sie mindestens einen Pflanzenextrakt enthält, der reich an Flavonoiden und / oder Tanninen ist.

**6.** Kosmetische Zusammensetzung nach einem der vorhergehenden Ansprüche, **dadurch gekennzeichnet, dass** sie einen oder mehrere Formulierhilfsstoffe oder Additive umfasst, wie etwa Weichmacher, Farbstoffe, filmbildende Wirkstoffe, Tenside, Duftstoffe, Konservierungsmittel, Emulgatoren, Öle, Glykole, Talg absorbierende Wirkstoffe, Vitamine und UV-Filter.

**7.** Kosmetische Verwendung eines Extrakts aus Bocoa prouacensis zur Bekämpfung der Hautalterung.

**8.** Verwendung eines Extrakts aus Bocoa prouacensis zur Herstellung einer kosmetischen oder dermatologischen Zusammensetzung für die Hautpflege.

**9.** Verwendung nach einem der Ansprüche 7 oder 8, **dadurch gekennzeichnet, dass** der Extrakt aus Bocoa prouacensis oder die Zusammensetzung, die ihn enthält, zur Bekämpfung der aktinischen Alterung bestimmt sind.

**10.** Verwendung nach einem der Ansprüche 7 oder 8, **dadurch gekennzeichnet, dass** der Extrakt aus Bocoa prouacensis oder die Zusammensetzung, die ihn enthält, zur Bekämpfung der intrinsischen Alterung bestimmt sind.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **ROBERT et al.** *J. Méd. Esth. et Chir. Derm.,* 2001, vol. 28, 27-35
- **BENOÎT et al.** *IFSCC Magazine,* 2000, vol. 3, 11-17
- **SHUSTER et al.** *Brit. J. Dermatol.,* 1975, vol. 93, 639-43
- **ALIREZAI ; MEYNADIER.** *Nouv. Dermatol.,* 1997, vol. 16, 41-47
- **PELLETIER-LEBON et al.** Cutaneous development, aging and repair. Fidia Research Series, Liviana Press, 1989, vol. 18, 341-345
- **ARCHILLA-MARCOS ; ROBERT.** *Clin. Physiol. Biochem.,* 1993, vol. 10, 86-91
- **LABAT-ROBERT et al.** *J. Photochem. Photobiol.,* 2000, vol. 57, 113-118